# EUROPEAN PATENT APPLICATION

(11) **EP 0 577 218 A1**
(43) Date of publication of application: **05.01.1994**
(21) Application number: 93201899.7
(22) Date of filing: 30.06.1993
(51) Int. Cl.: C07D 413/14, A61K 31/50

(54) **Heterocyclyl-phenoxyalkyl-piperidinylpyridazines as antiviral agents**

(30) Priority: 02.07.1992 US 909402
(71) Applicant: STERLING WINTHROP INC., New York, NY 10016 (US)
(72) Inventor: Diana, Guy Dominic, c/o Sterling Winthrop Inc., Rensselaer NY 12144 (US)
(74) Representative: Le Guen, Gérard

(57) **Abstract**

Compounds of formula 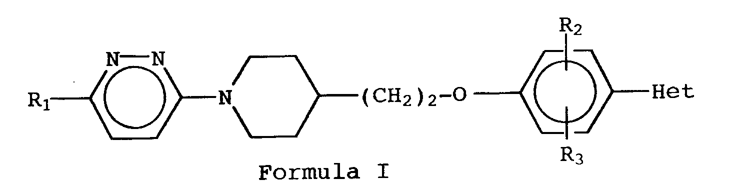
wherein Het is 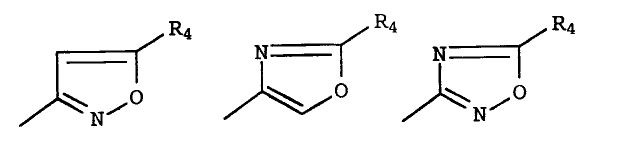
R₁ is a C₁-C₃ lower-alkyl;
R₂ and R₃ can each independently be hydrogen or fluorine;
R₄ is methyl or trifluoromethyl;
with the proviso that when Het is 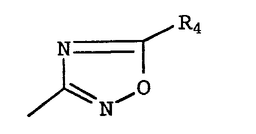
R₁ is methyl and R₂ is fluorine; and when Het is 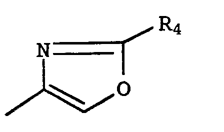
R₁ is methyl, R₂ is 2-fluoro, R₃ is hydrogen and
R₄ is methyl;
or pharmaceutically acceptable acid addition salts thereof, are useful as antiviral agents, particularly against picornaviruses.

## Description

This invention relates to certain novel heterocyclic substituted phenoxyalkyl-1-piperidinylpyridazines and to compositions and methods of use thereof as antiviral agents.

European Patent Publication No. 0320032 discloses compounds having the formula 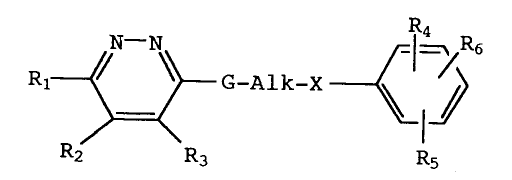
wherein:
R₁ is hydrogen, C₁₋₆ alkyl, halo, hydroxy, mercapto, trifluoromethyl, amino, mono or di-(C₁₋₆ alkyl)amino, cyano, C₁₋₆ alkyloxy, aryloxy, aryl-C₁₋₆ alkyloxy, C₁₋₆ alkylthio, arylthio, C₁₋₆ alkylsulfinyl, C₁₋₆ alkylsulfonyl, arylsulfinyl, arylsulfonyl, C₁₋₆ alkoxycarbonyl, C₁₋₆ alkylcarbonyl, or aryl;
R₂ and R₃ each independently are hydrogen or C₁₋₆ alkyl, or R₂ and R₃ combined may form a bivalent radical of formula -CH=CH-CH=CH-
Alk is an alkane chain of 0-6 carbons
G is a bivalent radical of formula 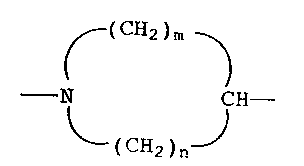
wherein
m is 2-3 carbons
n is 2-3 carbons
R₄, R₅ and R₆ are independently hydrogen, C₁₋₆ lower-alkyl, cyano, nitro, amino or halo.

X is O, S, NR₈ or a direct bond; said R₈ being hydrogen or C₁₋₆ alkyl.

The compounds are stated to have antiviral activity.

European Patent Publication No. 435381 discloses pyridazinamines of formula 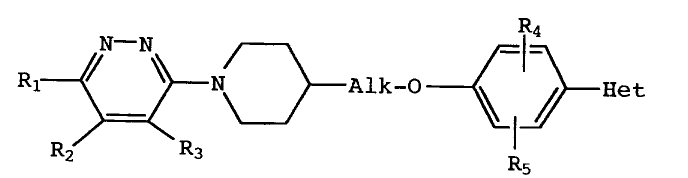
wherein
R₁ is hydrogen, C₁₋₄ alkyl, halo, hydroxy, trifluoromethyl, cyano, C₁₋₄ alkoxy, C₁₋₄ alkylthio, C₁₋₄ alkylsulfinyl, C₁₋₄ alkylsulfonyl, C₁₋₄ alkyloxycarbonyl, C₁₋₄ alkylcarbonyl or aryl;
R₂ and R₃ are hydrogen or C₁₋₄ alkyl;
Alk is C₁₋₄ alkanediyl;
R₄ and R₅ are hydrogen, C₁₋₄ alkyl or halo; and
Het is 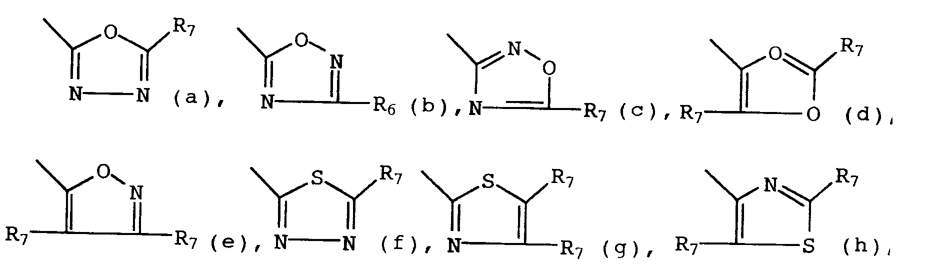
wherein
R₆ is hydrogen, C₁₋₆ alkyl; hydroxy-C₁₋₆ alkyl; C₃₋₆ cycloalkyl; aryl; aryl-C₁₋₄ alkyl; C₁₋₄ alkyloxy-C₁₋₄ alkyl; C₃₋₆ cycloalkyl-C₁₋₄ alkyl; trifluoromethyl or amino;
each R₇ independently is hydrogen; C₁₋₆ alkyl; C₃₋₆ cycloalkyl; aryl; aryl-C₁₋₄ alkyl; C₁₋₄ alkyloxy C₁₋₄ alkyl; C₃₋₆ cycloalkyl-C₁₋₄ alkyl or trifluoromethyl; and
each aryl independently is phenyl or phenyl substituted with 1 or 2 substituents each independently selected from halo, C₁₋₄ alkyl, trifluoromethyl, C₁₋₄ alkyloxy or hydroxy.

The compounds are stated to have antipicornaviral activity.

It has now been found that phenoxyalxylpiperidinylpyridazines wherein the phenoxy group is substituted with certain heterocycles are effective antiviral agents.

Accordingly the present invention relates to compounds of formula: 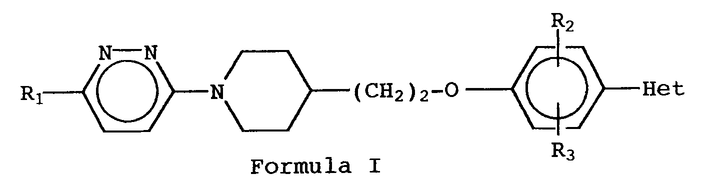
wherein
Het is 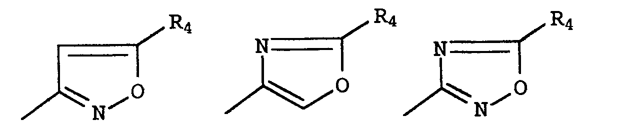
R₁ is a C₁-C₃ lower-alkyl;
R₂ and R₃ can each independently be hydrogen or fluorine;
R₄ is methyl or trifluoromethyl;
with the proviso that when Het is 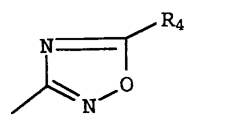
R₁ is methyl and R₂ is fluorine; and when Het is 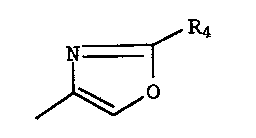
R₁ is methyl, R₂ is 2-fluoro, R₃ is hydrogen and
R₄ is methyl;
or pharmaceutically acceptable acid addition salts thereof.

Preferred compounds within the scope of Formula I are those wherein R₁ is methyl. When Het is 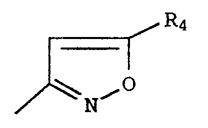
R₂ and R₃ are also preferably both hydrogen, most preferably when R₄ is methyl. When Het is 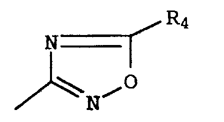
R₂ and R₃ are also preferably both fluorine, most preferably when R₄ is trifluoromethyl.

The invention also relates to compositions for combating viruses comprising an antivirally effective amount of a compound of Formula I with a suitable carrier or diluent, and to the use of a compound of Formula I for the preparation of a medicament for comnating a viral infection in a mammalian host.

The compounds of Formula I are prepared by the procedures outlined below:
4-(2-hydroxyethyl)piperidine and 3-halo-6-alkylpyridazine (V) 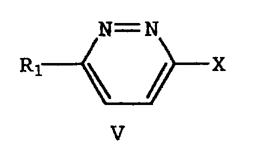
are reacted in the presence of a base (e.g. diisopropylethylamine) in a noninteracting solvent (e.g. N-methylpyrrolidine) preferably under reflux to give the 3-(4-(2-hydroxyethyl)-1-piperidinyl)-6-R₁-pyridazine (VI) 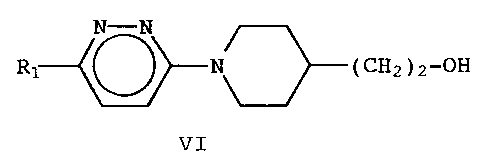
which in turn is reacted with triphenylphosphine (TPP) and 4-hydroxy-R₂-R₃-benzonitrile followed by diethyl azodicarboxylate (DEAD). The reaction is carried out in an inert solvent, e.g. methylene chloride or THF, at about 0°C to ambient temperature to give VII: 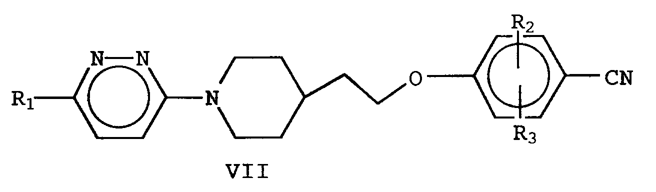
The above intermediate (VII) is reacted with hydroxylamine hydrochloride and sodium acetate in ethanol or aqueous ethanol, preferably under reflux, and the resulting product reacted with excess acetic anhydride at about 100 to 140°C to give a compound of Formula I wherein Het is 5-methyl-1,2,4-oxadiazol-3-yl, i.e. 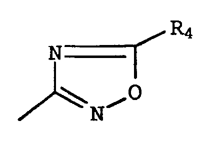
wherein R₄ is methyl.

The corresponding trifluoromethyl substituted compound (R₄=CF₃) can be prepared by substituting sodium trifluoroacetate for sodium acetate and trifluoroacetic anhydride for acetic anhydride.

Alternatively 4-methoxy-R₂-R₃-benzonitrile is reacted with hydroxylamine followed by trifluoroactic anhydride as described above to give 3-(4-hydroxy-R₂-R₃-phenyl)5-trifluoromethyl-1,2,4-oxadiazole. This oxadiazole is then reacted with intermediate VI in the presence of TPP and DEAD as previously described.

To prepare the 5-R₄-3-oxazolyl compounds of Formula I, 4-methoxy-R₂-R₃-acetophenone is reacted with lead tetraacetate to give the corresponding α-acetoxy acetophenone which is then reacted with ammonium acetate and acetic acid preferably under reflux. The methoxy group is cleaved with boron tribromide in an inert solvent, e.g. refluxing methylene chloride, giving 3-(4-hydroxy-R₂-R₃-phenyl)-5-R₄-oxazole VIII wherein R₄=CH₃. The corresponding compound in which R₄=CF₃ is obtained by substituting ammonium trifluoroacetate and trifluoroacetic acid for ammonium acetate and acetic acid. 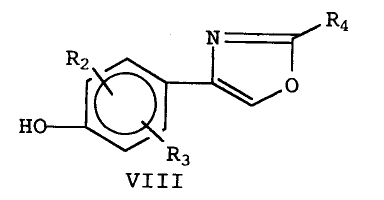
Intermediate VIII is then condensed with intermediate VI in the presence of TPP and DEAD, as described above for the preparation of VII, to give a compound of Formula I wherein Het is 5-R₄-3-oxazolyl, i.e. 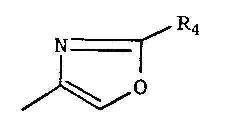
To prepare the compounds of Formula I wherein Het is 5-R₄-3-isoxazolyl, i.e. 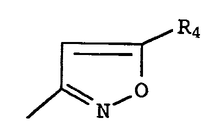
intermediate VI is reacted with a strong base in an aprotic solvent, e.g. sodium hydride in DMF, and the resulting sodium salt is reacted with an appropriate 3-(4-hydroxyphenyl)-5-R₄-isoxazole.

The starting materials, 4-(2-hydroxyethyl)piperidine, 3-halo-6-R₁-pyridazine, 4-methoxy-R₂-R₃-benzonitrile, and 4-hydroxy-R₂-R₃-benzonitrile belong to known classes of compounds and are commercially available or can be prepared by methods well known in the art.

The compounds of the invention are sufficiently basic to form acid-addition salts and are useful both in the free base form and the form of acid-addition salts, and both forms are within the purview of the invention. The acid-addition salts are in some cases a more convenient form for use, and in practice the use of the salt form inherently amounts to the use of the base form. The acids which can be used to prepare the acid-addition salts include preferably those which produce, when combined with the free base, medicinally acceptable salts, that is, salts whose anions are relatively innocuous to the animal organism in medicinal doses of the salts so that the beneficial properties inherent in the free base are not vitiated by side effects ascribable to the anions. Examples of appropriate acid-addition salts include the hydrochloride, hydrobromide, sulfate, acid sulfate, maleate, citrate, tartrate, methanesulfonate, p-toluenesulfonate, dodecyl sulfate, cyclohexanesulfamate, and the like. However, other appropriate medicinally acceptable salts within the scope of the invention are those derived from other mineral acids and organic acids.

The acid-addition salts of the basic compounds are prepared either by dissolving the free base in aqueous alcohol solution containing the appropriate acid and isolating the salt by evaporating the solution, or by reacting the free base and an acid in an organic solvent, in which case the salt separates directly, is precipitated with a second organic solvent, or can be obtained by concentration of the solution. Although medicinally acceptable salts of the basic compounds are preferred, all acid-addition salts are within the scope of the present invention. All acid-addition salts are useful as sources of the free base form even if the particular salt per se is desired only as an intermediate product as, for example, when the salt is formed only for purposes of purification or identification, or when it is used as an intermediate in preparing a medicinally acceptable salt by ion exchange procedures.

The structures of the compounds of the invention were established by the mode of synthesis, by elemental analysis, and by infrared, ultraviolet, nuclear magnetic resonance and mass spectroscopy. The course of the reactions and the identity and homogeneity of the products were assessed by thin layer chromatography (TLC) or gas-liquid chromatography (GLC).

The invention will now be described with reference to the following examples but is in no way limited thereto.

As described herein a noninteracting solvent can be N-methyl pyrrolidine (NMP), methylene chloride (CH₂Cl₂), tetrahydrofuran (THF), benzene or any other aprotic solvent that will not take part in the reaction. Unless otherwise specified all reactions are run in dried solvents under a nitrogen atmosphere. Certain reagents are specified by abbreviation: triphenylphosphine (TPP), triethylamine (TEA), diisopropylethylamine (DIPEA), and diethyl azodicarboxylate (DEAD). Ether is diethylether unless otherwise specified.

### Example 1

### (a) Compound of Formula VIII (R₂=hydrogen, R₃=2-fluoro, R₄=methyl).

1.68 g (10 mmol) 3-fluoro-4-methoxyacetophenone and 4.88 g (11 mmol) lead tetraacetate were dissolved in 10 ml benzene and refluxed. Ethylene glycol was used to quench the reaction. On standing, the resulting 3.19 g of yellow oil crystallized. 1.44 g (6.37 mmol) of the crystals and 2.31 g (30 mmol) of ammonium acetate were combined in 15 ml glacial acetic acid and refluxed for 4 hours. The product was poured into water, then basified. The aqueous layer was extracted twice with methylene chloride then concentrated to dryness and recrystallized from methylene chloride yielding 0.8 g of product, 0.59 g (2.85 mmol) of which was taken up in 25 ml methylene chloride and combined with 2 ml of 1M solution of boron tribromide in methylene chloride and refluxed for 30 minutes. The product was poured into water and basified. The aqueous layer was washed twice with methylene chloride. The organic layers were combined and washed with water, 1N HCl, and brine and evaporated to dryness. The product was recrystallized from methanol giving 0.09 g of 4-(2-fluoro-4-hydroxyphenyl)-2-methyloxazole

### (b) Compound of Formula I (R₁=CH₃,R₂=2-fluoro, R₃=hydrogen and Het=

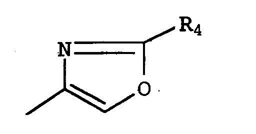

wherein R₄=CH₃)
0.46 g (2 mmol) of intermediate VIII was added to 15 ml methylene chloride, 1.05 g TPP and 1.63 g (3 mmol) 3-(4-(2-hydroxyethyl)-1-piperidinyl-6-methylpyridazine (Formula VI: R₁=CH₃) while cooling to 0°C. 0.63 ml (4 mmol) DEAD was added dropwise over 10 minutes. The solution came to room temperature and the organic phase was washed with water and evaporated. Recrystallization from ethyl acetate yielded 0.75 g of the compound of Formula I (m.p. 161-162°C.)

### Example 2

### (a) Compound of Formula IX

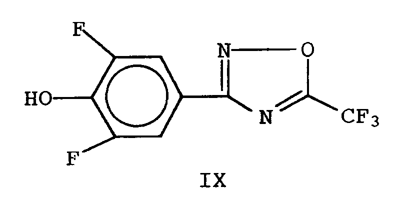

0.1 mol 3,5-difluoro-4-methoxybenzonitrile, 0.3 ml of hydroxylamine hydrochloride and 0.3 mol of potassium carbonate were added to 400 ml ethanol and refluxed overnight. The product was filtered and recrystallized from methanol giving 3.04 g of 3,5-difluoro-4-methoxybenzamide oxime. This product was dissolved in 5 ml pyridine and 5.6 ml trifluoroacetic anhydride was added dropwise at room temperature. Upon cooling the product solidified and was rinsed with water yielding 4.1 g of 3-(3,5-difluoro-4-hydroxyphenyl)-5-trifluoromethyl-1,2,4-oxadiazole (IX).

### (b) Compound of Formula I (R₁=CH₃, R₂ and R₃=3,5-difluoro and Het=

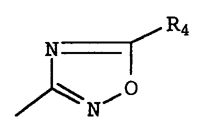

wherein R₄=CF₃)
0.1 g (0.38 mmol) of IX, 0.083 g (0.38 mmol) of 3-(4-(2-hydroxyethyl)-1-piperidinyl)-6-methylpyridazine (Formula VI: R₁=CH₃), 0.199 g TPP and 10 ml THF were combined and cooled in an ice bath. 120 µl (0.75 mmol) DEAD was added with stirring. The mixture was worked up and extracted as described above, then recrystallized from ethyl acetate giving 64 mg of the compound of Formula I (m.p. 94-95.5°C).

### Example 3

### Compound of Formula I (R₁=CH₃, R₂=R₃=hydrogen and Het =

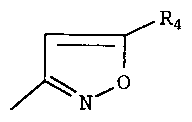

wherein R₄=CH₃)
To 100 g 80% sulfuric acid 10.0 g (42.7 mmol) 5,N-dimethyl-3-(4-hydroxyphenyl)-4-isoxazolecarboxamide was added and the mixture heated to just below reflux temperature for 3 hours. The solution was added to ice and the resulting white solid was filtered off and washed with water. The solid was recrystallized from hexane after treatment with charcoal giving a 91% yield of 3-(4-hydroxyphenyl)-5-methylisoxazole.

0.127 g (5 mmol) sodium hydride was dissolved in 8 ml dimethylformamide. 1.11 g (5 mmol) of 3-(4-(2-hydroxyethyl)-1-piperidinyl-6-methylpyridazine (Formula VI: R₁=CH₃) was added and stirred for 10 minutes at room temperature. To this mixture 0.89 g 3-(4-hydroxyphenyl)-5-methylisoxazole was added. The mixture was refluxed for 17 hours. Upon cooling water was added, then saturated salt. The product was extracted with methylene chloride. The extract was washed twice with water and dried over MgSO₄. The extract was concentrated in vacuo and the resulting oil was treated with charcoal and recrystallized from acetonitrile giving the compound of Formula I (m.p. 141-146°C).

Biological evaluation of compounds of Formula I shows that they possess antiviral activity. They are useful in inhibiting virus replication in vitro and are primarily active against picornaviruses, and especially numerous strains of rhinoviruses. The in vitro testing of the compounds of the invention against picornaviruses showed that viral replication was inhibited at minimum inhibitory concentrations (MIC) ranging from about 0.01 to about 5 micrograms per milliliter.

The MIC values were determined by a standard plaque reduction assay as follows: HeLa (Ohio) cells in monolayers were infected at a concentration of virus to give approximately 80 plaques per monolayer in the virus control (no drug present). The compound to be tested was serially diluted and included in the agarmedium overlay and in some cases, during the adsorption period as well. The MIC was determined to be that concentration of compound which reduced the number of plaques by 50% with respect to the untreated virus control.

In the standard test procedure, the compounds were tested against a panel of fifteen human rhinovirus (HRV) serotypes, namely HRV-2, -1A,, 1B, -6, -14, -21, -22, -15, -25, -30, -50, -67, -89, -86 and -41. The MIC value for each rhinovirus serotype was determined, and the efficacy of each compound weight average MIC vs type was determined in terms of MIC₅₀ and MIC₈₀ values, which is the concentration of the compound required to inhibit 50% and 80%, respectively, of the tested serotypes. MIC values are weighted averages for number of serotypes tested.

The following Table gives the testing results with the compounds of Formula I. The number of serotypes (N) is indicated after the MIC₈₀ figure.

| Example No. | MIC₅₀ | MIC₈₀ | N |
|---|---|---|---|
| Ex. 1 | 6.631 | .025 | 15 |
| Ex. 2 | 7.172 | .201 | 14 |
| Ex. 3 | 33.254 | 99.0 | 12 |

The antiviral compositions are formulated for use by preparing a dilute solution or suspension in a pharmaceutically acceptable aqueous, organic or aqueous/organic medium for topical or parenteral administration by intravenous or intramuscular injection, or for intranasal or ophthalmic application; or are prepared in tablet, capsule, or aqueous suspension form with conventional excipients for oral administration.

## Claims

1. A compound of the formula 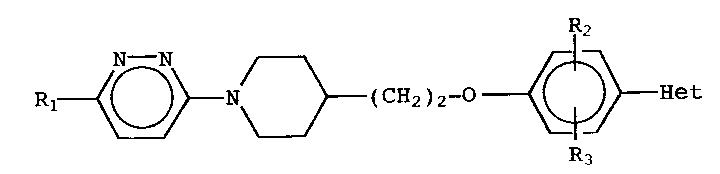 wherein
Het is 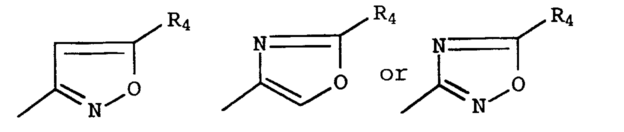 R₁ is a C₁-C₃ lower-alkyl;
R₂ and R₃ can each independently be hydrogen or fluorine;
R₄ is methyl or trifluoromethyl;
with the proviso that when Het is 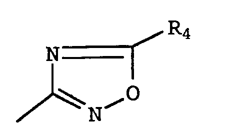 R₁ is methyl and R₂ is fluorine; and when Het is 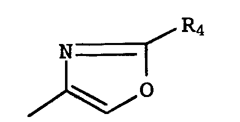 R₁ is methyl, R₂ is 2-fluoro, R₃ is hydrogen and
R₄ is methyl;
or pharmaceutically acceptable acid addition salts thereof.

2. A compound as claimed in claim 1 wherein R₁ is methyl.

3. A compound as claimed in claim 2 wherein R₂ and R₃ are both hydrogen and Het is

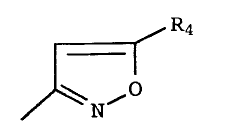

4. A compound as claimed in claim 3 wherein R₄ is methyl.

5. A compound as claimed in claim 2 wherein R₂ and R₃ are both fluorine and Het is

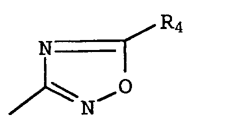

6. A compound as claimed in claim 5 wherein R₄ is trifluoromethyl.

7. A composition comprising a pharmaceutical carrier and as an active ingredient a compound as claimed in any one of the preceding claims.

8. The use of a compound as claimed in any one of claims 1 to 6 for the preparation of a medicament for combating an infection in a mammalian host.
